# EUROPEAN PATENT APPLICATION

(11) **EP 4 834 922 A1**
(43) Date of publication of application: **04.11.2026**
(21) Application number: 24911357.2
(22) Date of filing: 26.12.2024
(51) Int. Cl.: C07K 14/54, A61K 38/20, C12N 15/62

(54) **RECOMBINANT IL-27 PROTEIN AND PREPARATION METHOD THEREFOR**

(30) Priority: 27.12.2023 WO PCT/CN2023/142414
(71) Applicant: Wuxi Biologics Ireland Limited, Dundalk, Co Louth A91 X56F (IE)
(72) Inventor: LU, Mengjie, Shanghai 200131 (CN); WU, Jiansheng, Shanghai 200131 (CN); ZENG, Deyang, Shanghai 200131 (CN)
(74) Representative: Modiano, Gabriella Diana
(86) International application number: PCT/CN2024/142834
(87) International publication number: WO 2025/140436

(57) **Abstract**

Disclosed is a p28 subunit of a recombinant IL-27 protein, characterized in that it comprises an amino acid sequence selected from any one of SEQ ID NO: 1, 3, 5, 7, 9, 11, 13, 15, 17, 19, 21, 23, and 25. Disclosed is an EBI3 subunit of a recombinant IL-27 protein, characterized in that it comprises an amino acid sequence selected from any one of SEQ ID NO: 2, 4, 6, 8, 10, 12, 14, 16, 18, 20, and 22. Also disclosed are a recombinant IL-27 protein comprising said two subunits, and preparation and screening methods thereof.

## Description

### FIELD OF THE INVENTION

The present invention relates to the field of recombinant protein expression. Specifically, the present invention relates to a recombinant IL-27 protein, modified subunits thereof, and a method for preparing the recombinant IL-27 protein.

### BACKGROUND

IL-27 is a heterodimeric cytokine composed of p28 and EBI3, mainly expressed in macrophages, inflammatory monocytes, microglia and dendritic cells, and is also expressed in plasma cells, endothelial cells and epithelial cells. IL-27R, the receptor of IL-27, is composed of cytokine binding protein α subunit (IL-27Rα/Wsx-1) and signal transduction protein β (gp130) subunit. IL-27Rα has highest expression level in T cells, and also has a relatively high expression level in B cells and macrophages.

IL-27 is a pleiotropic cytokine. As an immune activator, IL-27 can prevent progression of cancers and infectious diseases; while as an immunomodulator, IL-27 can inhibit onset and progression of autoimmune diseases. IL-27 is an attractive candidate target for tumor immunotherapy, treatment of infectious diseases and autoimmune diseases.

Currently, recombinant human IL-27 protein is supplied by multiple vendors on the market, such as R&D Systems (Recombinant human IL-27 protein 2526-IL-010; R&D Systems (rndsystems.com)) and Sino Biological (Recombinant human IL-27 protein, stable CHO cells, Cat. #: 10076-H08S; Sino Biological), among others. However, all these products are expressed as a single protein by linking the p28 and EBI3 subunits via a (GGS)n linker, which suffers from severe protein aggregation. And after multi-step purification, the product purity is only greater than 90%, which cannot meet clinical needs. To increase the half-life, some also link it to the N-terminus of Fc, and express and purify it as an Fc fusion protein. However, the aggregation of the molecule is very severe, and the monomer yield is extremely low, which cannot be produced on a large scale.

IL-27 molecules, no matter in any form, will form severe aggregates, thereby limiting the production and clinical application thereof.

Therefore, there is a need in the art for a method of stably producing IL-27 that overcomes the formation of IL-27 molecular aggregates.

### SUMMARY OF THE INVENTION

One object of the present disclosure is to solve the problem of aggregate formation of IL-27 molecules during the production process.

The inventors, in view of the severe aggregation problem of IL-27 protein, introduced disulfide bonds into the IL-27 protein to reduce aggregation, thereby improving the yield and clinical application potential of the protein.

In one aspect of the present disclosure, provided is a method for preparing a recombinant IL-27 protein, comprising:
introducing a cysteine substitution into a p28 subunit of a recombinant IL-27 protein, wherein the p28 subunit having the amino acid sequence of 
MGQTAGDLGWRLSLLLLPLLLVQAGVWGFPRPPGRPQLSLQELRREFTVSLHLARKLLS
EVRGQAHRFAESHLPGVNLYLLPLGEQLPDVSLTFQAWRRLSDPERLCFISTTLQPFHAL
LGGLGTQGRWTNMERMQLWAMRLDLRDLQRHLRFQVLAAGFNLPEEEEEEEEEEEEE
RKGLLPGALGSALQGPAQVSWPQLLSTYRLLHSLELVLSRAVRELLLLSKAGHSVWPLG
FPTLSPQP (SEQ ID NO: 26),
introducing a cysteine substitution into an EBI3 subunit of a recombinant IL-27 protein, wherein the EBI3 subunit having the amino acid sequence of 
MTPQLLLALVLWASCPPCSGRKGPPAALTLPRVQCRASRYPIAVDCSWTLPPAPNSTSPV
SFIATYRLGMAARGHSWPCLQQTPTSTSCTITDVQLFSMAPYVLNVTAVHPWGSSSSFV
PFITEHIIKPDPPEGVRLSPLAERQLQVQWEPPGSWPFPEIFSLKYWIRYKRQGAARFHRV
GPIEATSFILRAVRPRARYYVQVAAQDLTDYGELSDWSLPATATMSLGK (SEQ ID NO:
27);
synthesizing the two modified subunits artificially and assembling them to obtain a recombinant IL-27 protein; or alternatively, obtaining coding sequences of the subunits, transfecting them into an expression vector, and expressing them in cells to obtain a recombinant IL-27 protein; and
determining the activity of the recombinant IL-27 protein and comparing the activity with that of a wild-type IL-27, thereby obtaining a recombinant IL-27 protein having an activity greater than that of the wild-type IL-27.

In one embodiment of this aspect, the activity of the recombinant IL-27 protein is preferably tested by comparing the molecular weight of the recombinant IL-27 protein with that of wild-type IL-27, preferably by chromatography, and more preferably by liquid chromatography-mass spectrometry (LC-MS); by a immunological assay, preferably an enzyme-linked immunosorbent assay (ELISA); or by an *in vitro* cell assay, preferably a cytopathic effect inhibition assay.

In another aspect of the present disclosure, a recombinant p28 subunit of a IL-27 protein is provided, characterized in that it comprises an amino acid sequence selected from any one of SEQ ID NO: 1 (p28_S91C), SEQ ID NO: 3 (p28_D89C), SEQ ID NO: 5 (p28_L83C), SEQ ID NO: 7 (p28_Q86C), SEQ ID NO: 9 (p28_F94C), SEQ ID NO: 11 (p28_Q95C), SEQ ID NO: 13 (p28_L80C), SEQ ID NO: 15 (p28_P82C), SEQ ID NO: 17 (p28_G84C), SEQ ID NO: 19 (p28_E85C), SEQ ID NO: 21 (p28_L87C), SEQ ID NO: 23 (p28_L81C), and SEQ ID NO: 25 (p28_Y79C).

In a third aspect of the present disclosure, an recombinant EBI3 subunit of a IL-27 protein is provided, characterized in that it comprises an amino acid sequence selected from any one of SEQ ID NO:2 (EBI3_S98C), SEQ ID NO:4 (EBI3_M99C), SEQ ID NO:6 (EBI3_Q95C), SEQ ID NO:8 (EBI3_P41C), SEQ ID NO:10 (EBI3_L96C), SEQ ID NO:12 (EBI3_D93C), SEQ ID NO:14 (EBI3_G69C), SEQ ID NO:16 (EBI3_M70C), SEQ ID NO:18 (EBI3_A100C), SEQ ID NO:20 (EBI3_P101C), and SEQ ID NO:22 (EBI3_P120C).

In a fourth aspect of the present invention, a recombinant IL-27 protein is provided, characterized in that it comprises a p28 subunit and an EBI3 subunit; wherein the p28 subunit comprises an amino acid sequence selected from any one of SEQ ID NO: 1, 3, 5, 7, 9, 11, 13, 15, 17, 19, 21, 23 and 25, and the EBI3 subunit comprises an amino acid sequence selected from any one of SEQ ID NO: 2, 4, 6, 8, 10, 12, 14, 16, 18, 20 and 22.

In a preferred embodiment of this aspect, the p28 subunit and EBI3 subunit are selected from the following combinations:
SEQ ID NO: 1 and SEQ ID NO: 2 (p28_S91C/EBI3_S98C), SEQ ID NO: 3 and SEQ ID NO: 2 (p28_D89C/EBI3_S98C), SEQ ID NO: 3 and SEQ ID NO: 4 (p28_D89C/EBI3_M99C), SEQ ID NO: 5 and SEQ ID NO: 4 (p28_L83C/EBI3_M99C), SEQ ID NO: 7 and SEQ ID NO: 4 (p28_Q86C/EBI3_M99C),SEQ ID NO: 17 and SEQ ID NO: 4 (p28_G84C/EBI3_M99C), SEQ ID NO: 19 and SEQ ID NO: 4 (p28_E85C/EBI3_M99C), SEQ ID NO: 21 and SEQ ID NO: 4 (p28_L87C/EBI3_M99C), SEQ ID NO: 1 and SEQ ID NO: 6 (p28_L87C/EBI3_M99C),SEQ ID NO: 9 and SEQ ID NO: 8 (p28_F94C/EBI3_P41C), SEQ ID NO: 9 and SEQ ID NO: 10 (p28_F94C/EBI3_L96C), SEQ ID NO: 11 and SEQ ID NO: 12 (p28_Q95C/EBI3_D93C),SEQ ID NO: 5 and SEQ ID NO: 14 (p28_L83C/EBI3_ G69C), SEQ ID NO: 5 and SEQ ID NO: 16 (p28_L83C/EBI3_M70C), SEQ ID NO: 13 and SEQ ID NO: 16 (p28_L80C/EBI3_M70C), SEQ ID NO: 15 and SEQ ID NO: 16 (p28_P82C/EBI3_M70C),SEQ ID NO: 5 and SEQ ID NO: 18 (p28_L83C/EBI3_A100C), SEQ ID NO: 23 and SEQ ID NO: 18 (p28_L81C/EBI3_A100C), SEQ ID NO: 13 and SEQ ID NO: 20 (p28_L80C/EBI3_P101C), SEQ ID NO: 13 and SEQ ID NO: 22 (p28_L80C/EBI3_P120C), SEQ ID NO: 25 and SEQ ID NO: 22 (p28_Y79C/EBI3_P120C).

The sequences described herein are as defined in the computer-readable Sequence Listing of the specification. The content thereof is hereby incorporated into the specification.

In one embodiment of the present disclosure, the N-terminals of the amino acid sequences of the p28 subunit and EBI3 subunit of the recombinant IL-27 protein may further comprise a signal peptide. In a preferred embodiment, the signal peptide has an amino acid sequence of MGWSCIILFLVATATGVHS (SEQ ID NO: 24).

In another aspect of the present disclosure, further provided is an isolated nucleic acid molecule encoding the recombinant IL-27 protein of the present disclosure or one or more subunits thereof. The nucleic acid is preferably DNA.

In still another aspect of the present disclosure, further provided is an expression vector comprising and expressing the nucleic acid molecule as recited in claim 6. The expression vector may be a transient or constitutive transfection vector, and preferably a transient transfection vector. The vector may be selected from plasmids or viral vectors. The vector is preferably a mammalian vector.

In another aspect of the present disclosure, the disclosure further comprises an isolated host cell comprising the expression vector of the present disclosure. Preferably, the cell is a mammalian cell, and more preferably, a CHO cell. In another aspect of the present disclosure, the cell of the present disclosure may be a human cell.

### BRIEF DESCRIPTION OF THE FIGURES

Figure 1 depicts the SDS-PAGE gel analysis diagrams of recombinant IL-27 proteins 1-9.
Figure 2 depicts the HPLC results of recombinant IL-27 proteins 1-9.
Figures 3 and 4 show the test results of the binding activities of recombinant IL-27 proteins 1-9 to human IL27RA-Fc.
Figures 5 and 6 show concentration-dependent changes in HepG2 cell absorbance following incubation with recombinant IL-27 proteins 1-9 and subsequent viral challenge.
Figure 7 depicts the SDS-PAGE gel analysis diagrams of recombinant IL-27 proteins 10-21.

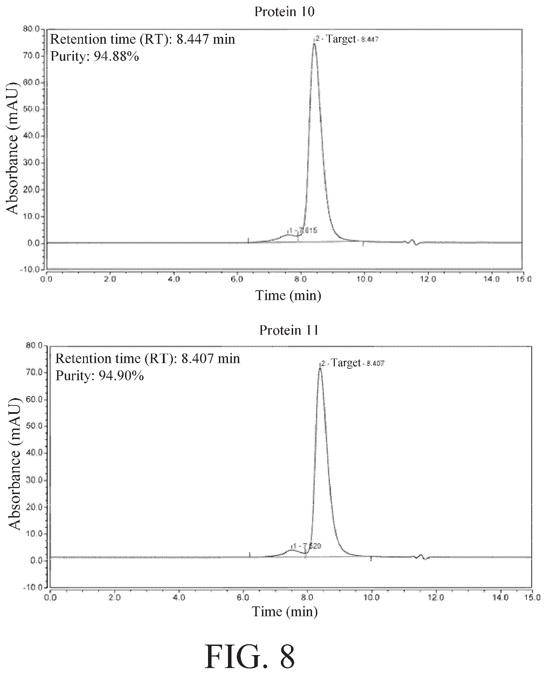

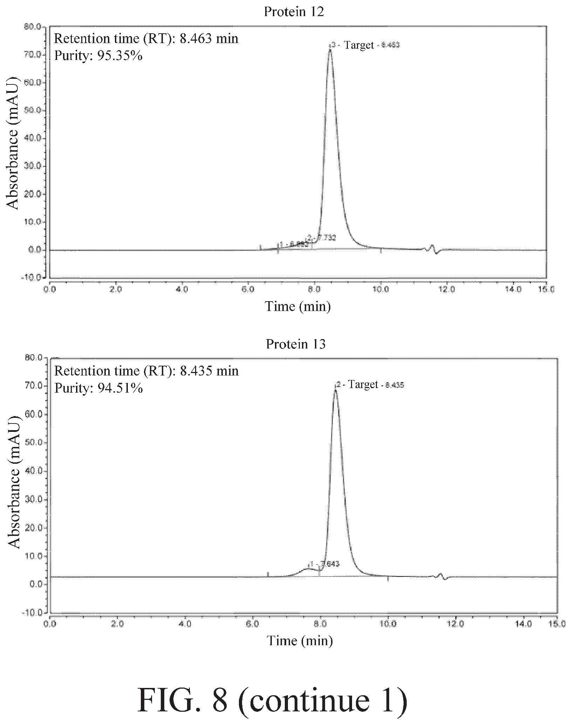

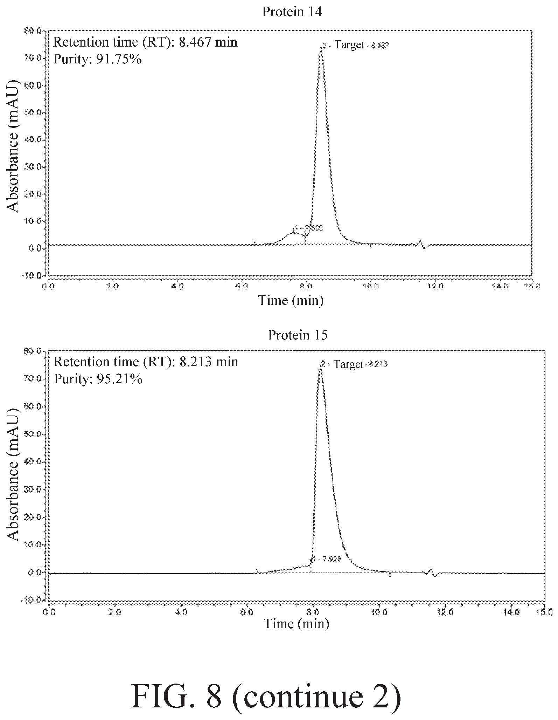

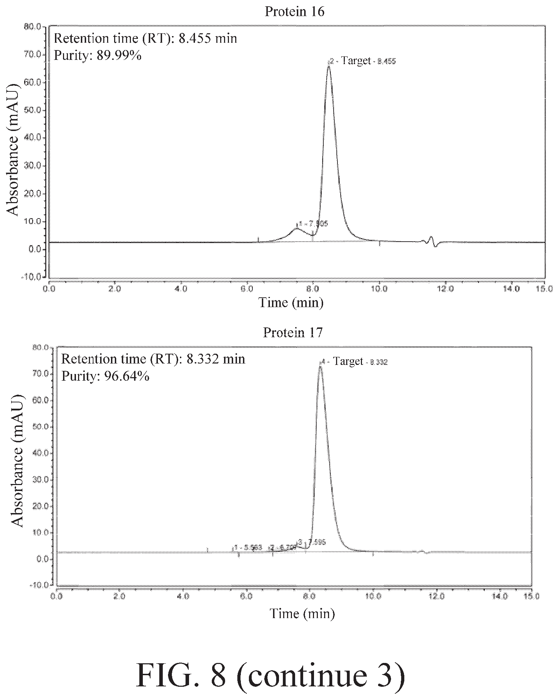

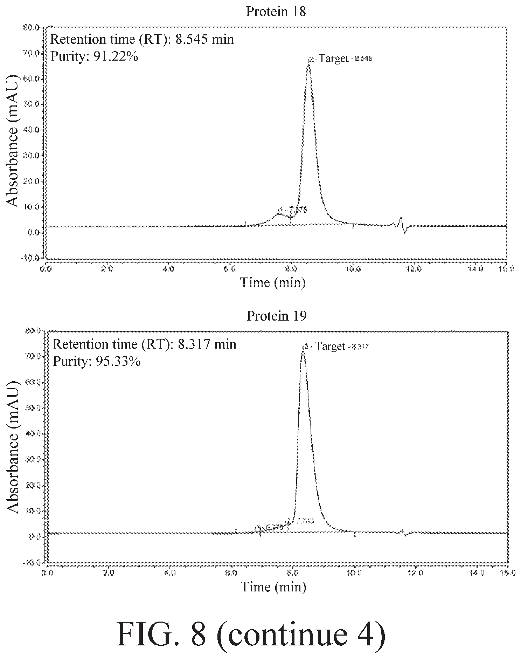

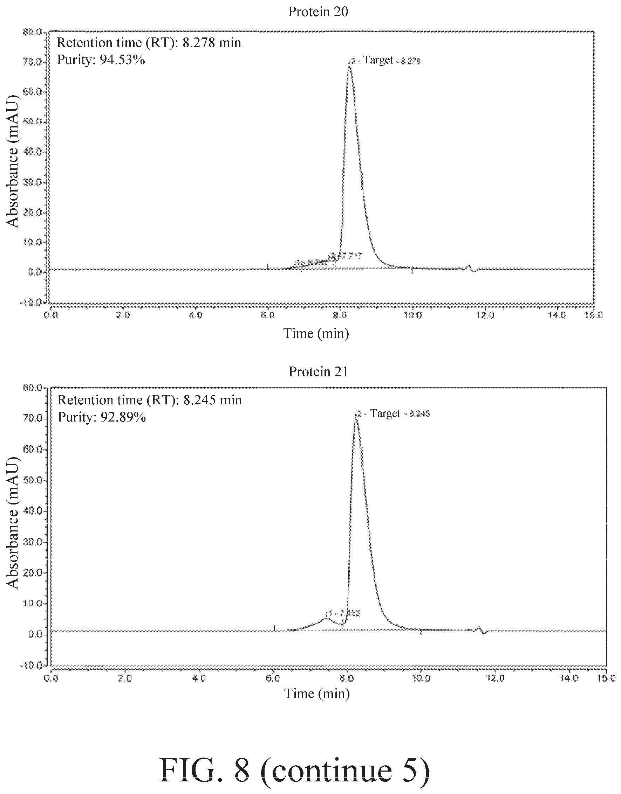

Figure 8 depicts the HPLC results of recombinant IL-27 proteins 10-21.
Figure 9 shows the test results of the binding activities of recombinant IL-27 proteins 10-21 to human IL27RA-Fc.
Figure 10 shows concentration-dependent changes in HepG2 cell absorbance following incubation with recombinant IL-27 protein 10-21 and subsequent virus challenge.

### DETAILED DESCRIPTION OF THE INVENTION

The examples discussed below are only intended to illustrate the present invention, and shall not be deemed as limiting the present invention in any way. These examples are not intended to represent that the following experiments are all or the only experiments carried out. Efforts have been made to ensure the accuracy of the quantities used (such as amounts, temperatures, etc.), but some experimental errors and deviations shall be considered. Unless otherwise specified, parts are parts by weight, molecular weights are average molecular weights, temperatures are in degrees Celsius, and pressures are at or near atmospheric pressure.

### Definitions

As used herein, unless the context clearly provides otherwise, singular forms starting with "a", "an" and "the" include plural references. Likewise, the terms "a" (or "an"), "one or more" and "at least one" may be used interchangeably herein.

As used herein, the terms "about", "approximately" or "roughly", when preceding a numerical value, refer to a range defined by approximate values within 1%, 2%, 3%, 4%, 5%, 10% or more of the specified value.

In the present disclosure, one or more features in one embodiment may be combined with any one or more features in another embodiment without departing from the spirit and concept of the present invention.

In the present disclosure, unless otherwise specified, all ranges, including ranges defined as between two specified end values, include the specified end values. For example, a range between 1 and 10 refers to a range between 1 and 10 (including 1 and 10).

In the present disclosure, when a duration, period or interval is expressed in days, or a time point is stated as a certain day, it means that the duration or time point is calculated or distinguished on a daily basis, and the numerical value thereof is not required to be a multiple of 24 hours.

In the present disclosure, unless otherwise specified, the technical and scientific terms used have the same meanings as those commonly understood by a person of ordinary skill in the art to which the present invention pertains. All publications, patent applications, patents, and other references mentioned herein are incorporated by reference in their entirety to form the disclosure thereof. The citations are only used to illustrate the technical level of those skilled in the art and are not admitted as prior art to the present invention.

As described herein, "human recombinant IL-27", "human recombinant IL-27 protein" or "rhIL-27" refers to a mutant IL-27 protein that is genetically engineered and retains the biological activity of IL-27. The p28 sequence subunit of wild-type human IL-27 protein has the amino acid sequence of
MGQTAGDLGWRLSLLLLPLLLVQAGVWGFPRPPGRPQLSLQELRREFTVSLHLARKLLS EVRGQAHRFAESHLPGVNLYLLPLGEQLPDVSLTFQAWRRLSDPERLCFISTTLQPFHAL LGGLGTQGRWTNMERMQLWAMRLDLRDLQRHLRFQVLAAGFNLPEEEEEEEEEEEEE RKGLLPGALGSALQGPAQVSWPQLLSTYRLLHSLELVLSRAVRELLLLSKAGHSVWPLG FPTLSPQP(SEQ ID NO:26), wherein the underlined portion is the native signal peptide sequence thereof. The wild-type IL-27 EBI3 sequence has the amino acid sequence of MTPQLLLALVLWASCPPCSGRKGPPAALTLPRVQCRASRYPIAVDCSWTLPPAPNSTSPV SFIATYRLGMAARGHSWPCLQQTPTSTSCTITDVQLFSMAPYVLNVTAVHPWGSSSSFV PFITEHIIKPDPPEGVRLSPLAERQLQVQWEPPGSWPFPEIFSLKYWIRYKRQGAARFHRV GPIEATSFILRAVRPRARYYVQVAAQDLTDYGELSDWSLPATATMSLGK (SEQ ID NO: 27), wherein the underlined portion is the native signal peptide sequence thereof. In the present disclosure, a cysteine mutation is introduced at suitable positions on the interaction interface of the two subunits of IL-27, p28 and EBI3, respectively, and the disulfide bond(s) formed therebetween stabilizes the interaction between p28 and EBI3, thereby reducing aggregation. It should be understood that, except for the specific cysteine mutation modifications, the remaining sequence(s) may have at least 70%, at least 75%, at least 80%, at least 85%, at least 90%, at least 95%, at least 99% or 100% sequence identity to the sequence of a naturally-derived mature interleukin 27, while retaining the biological activity thereof. Interleukin 27 protein that can be produced according to the methods of the present disclosure includes cysteine mutation-modified forms of recombinant proteins in various functional forms. Commercially available products include, for example, those from R&D Systems (Recombinant human IL-27 protein 2526-IL-010; R&D Systems (rndsystems.com)), Sino Biological (Recombinant human IL-27 protein, CHO stable cell, Cat. #: 10076-H08S; Sino Biological), etc. The amino acid sequences of the p28 subunit and EBI3 subunit of the recombinant IL-27 protein used in the Examples are set forth in any one of SEQ ID NO: 1, 3, 5, 7, 9, 11, 13, 15, 17, 19, 21, 23, 25 and any one of SEQ ID NO: 2, 4, 6, 8, 10, 12, 14, 16, 18, 20, 22, respectively. Conservative variants, homologous substitutes, or functional fragments thereof may also be used.

The mammalian cells described herein may be derived from a variety of mammalian sources, including but not limited to primates (e.g., humans and non-human primates such as chimpanzees, baboons or monkeys), dogs, cats, pigs, sheep, rabbit, mice and rats. In some cases, the mammal may be a human.

Preferred mammalian cells are CHO cells and HEK293 cells. Other mammalian cells may also be used, for example, NS0 and the like.

The term "expression vector" as used herein refers to a nucleic acid molecule that, after entering a host cell, is capable of directing the expression of a gene operably linked thereto. One type of vector is a "plasmid", which refers to a circular double-stranded DNA loop into which additional DNA segments can be ligated. Another type of vector is a viral vector, wherein additional DNA segments can be ligated into the viral genome. Certain vectors are capable of autonomous replication in a host cell into which they are introduced (e.g., bacterial vectors with a bacterial origin of replication and episomal mammalian vectors). Other vectors (e.g., non-episomal mammalian vectors), when introduced into a host cell, can be integrated into the genome of the host cell, and thus replicate together with the host genome. Standard techniques can be used for recombinant DNA, oligonucleotide synthesis, as well as tissue culture and transformation (e.g., electroporation, lipofection). Enzymatic reactions and purification techniques may be performed according to the manufacturer's instructions, as commonly practiced in the art, or as described herein. The aforementioned techniques and procedures can generally be performed according to conventional methods well known in the art and as described in various general and more specific references that are cited and discussed throughout the specification of the present disclosure.

A pharmaceutically acceptable composition of the present disclosure comprises one or more forms of the recombinant IL-27 protein described herein, or a combination thereof, a nucleic acid molecule encoding the same, a recombinant expression vector, a host cells, and a pharmaceutically acceptable excipient, such as a carrier or a diluent.

An advantage of the present disclosure is that, based on professional knowledge and experience in structural biology and protein engineering, twenty-one (21) pairs of cysteine mutation combinations were designed at the interaction interface of the two subunits, p28 and EBI3, of the human IL-27 protein, respectively, to form disulfide bonds that stabilize the interaction therebetween, thereby reducing protein aggregation arising from weak non-covalent interaction therebetween. This approach substantially reduces aggregate formation during the production of IL-27 protein, rendering the production of the IL-27 protein more economical and yielding products of superior quality.

### EXEMPLES

### Example 1 Expression and Purification of Recombinant Human IL-27 Protein

Recombinant human IL-27 proteins 1-9 were expressed and purified using the same method described below.

### 1. Materials and Equipment

The equipment and materials used in this study are shown in Table 1 and Table 2 below.

**Table 1 Equipment**

| Equipment Name | Equipment Manufacturer | Model |
|---|---|---|
| Centrifuge | Beckman Coulter, USA | Avanti J-26S XP |
| LC-MASS | Agilent Technologies | 6224 TOF LC/MS |
| AKTA Pure M150 | GE, USA | AKTA Pure M150 |
| Nanodrop 2000 | Thermo | Nanodrop 2000 |
| HPLC | Thermo, USA | Vanquish^{™} Duo UHPLC |
| SDS-PAGE | Tanon | EPS600 |
| SEC-column | TSKgel, JAPAN | G3000SWxl |
| Shaker | KUHNER | SMX1604C |
| Biological safety cabinet | SUZHOU ANTAI AIRTECH | BCS-1600IIA2 |
| CO₂ incubator | Thermo | 3111 |
| Inverted microscope | Olympus | XDS-1B |
| Single-channel pipette | RAININ, THERMO | 1000ul-10ul |
| 12-channel pipette | Thermo | 50ul |
| Electric pipette | BRAND | 16G10270 |
| Vortex mixer | Thermo | LP Vortex Mixer |
| Low-speed centrifuge | AHUI USTC Zonkia Scientific Instruments Co.,Ltd | SC-3612 |

**Table 2: Reagents and Equipment**

| Name | Supplier | Cat. #: |
|---|---|---|
| Capsule filter | Cobetter | L05HHSAFSS1P |
| Transfection medium | Wuxi Biologics Platform Medium | NA |
| Transfection feed medium | Wuxi Biologics Platform Medium | NA |
| Cell culture shake flask | Nest | 787011 |
| CHO K1 | | |
| M07E | ACCEGEN BIOTECHNOLOGY | ABC-TC1313 |
| WST8 | ABCAM | ab228254 |
| Ni-Excel | Cytiva | 17371202 |
| Q HP | Cytiva | 17115301 |
| NaOH | Hunan Er-kang Pharmaceutical | NA |
| NaCl | Jiangsu Province Qinfen Pharmaceutical | NA |
| Tromethamine (Tris base) | Suhua Pharmaceutical | NA |
| Triton X-100 | SIGMA | 2315025 |
| Triton X-114 | SIGMA | 2315025 |
| Potassium dihydrogen phosphate (KH₂PO₄) | Hunan Jiudian Pharmaceutical | NA |
| Dipotassium hydrogen phosphate trihydrate (K₂HPO₄·3H₂O) | Hunan Jiudian Pharmaceutical | NA |
| Potassium chloride (KCl) | Hushi | NA |
| Disodium hydrogen phosphate dodecahydrate (Na₂HPO₄·12H₂O) | Hushi | NA |
| IL27 Positive control | Sino Biological Inc. | 10076-H08S |
| Bovine Serum Albumin (BSA) | Sigma aldrich | SRE0098-500G |

### 2. Methods:

### 1) Construction methods for transient transfection plasmids:

Each protein of the present disclosure comprises two subunits, p28 and EBI3. The p28 gene (e.g., the corresponding p28 sequence in each of Proteins 1-21) and EBI3 gene (e.g., the corresponding EBI3 sequence in each of Proteins 1-21), which serve as expression constructs, were synthesized via whole-gene synthesis. And the subunit combinations are shown below.

| | | | | |
|---|---|---|---|---|
| Protein No. | P28 subunit | SEQ ID NO: | EBI3 subunit | SEQ ID NO: |
| 1 | p28_S91C | 1 | EBI3_S98C | 2 |
| 2 | p28_D89C | 3 | EBI3_M99C | 4 |
| 3 | p28_L83C | 5 | EBI3_M99C | 4 |
| 4 | p28_Q86C | 7 | EBI3_M99C | 4 |
| 5 | p28_D89C | 3 | EBI3_S98C | 2 |
| 6 | p28_S91C | 1 | EBI3_Q95C | 6 |
| 7 | p28_F94C | 9 | EBI3_P41C | 8 |
| 8 | p28_F94C | 9 | EBI3_L96C | 10 |
| 9 | p28_Q95C | 11 | EBI3_D93C | 12 |
| 10 | p28_ L83C | 5 | EBI3_ G69C | 14 |
| 11 | p28_ L80C | 13 | EBI3_ M70C | 16 |
| 12 | p28_ P82C | 15 | EBI3_ M70C | 16 |
| 13 | p28_L83C | 5 | EBI3_ M70C | 16 |
| 14 | p28_G84C | 17 | EBI3_M99C | 4 |
| 15 | p28_ E85C | 19 | EBI3_ M99C | 4 |
| 16 | p28_ L87C | 21 | EBI3_ M99C | 4 |
| 17 | p28_ L83C | 5 | EBI3_ A100C | 18 |
| 18 | p28_ L81C | 23 | EBI3_ A100C | 18 |
| 19 | p28_ L80C | 13 | EBI3_ P101C | 20 |
| 20 | p28_ L80C | 13 | EBI3_ P120C | 22 |
| 21 | p28_ Y79C | 25 | EBI3_ P120C | 22 |

The sequences were separately constructed into the expression vector pWX4.1 (provided by WuXi Biologics (Shanghai) Co., Ltd.; the vector backbone is a commercially available pTT5 vector backbone) to obtain recombinant human expression vectors pWX4.1/p28 and pWX4.1/EBI3, which were subsequently transformed into *E. coli* BL21/DH5α host strains, respectively. The above bacterial cultures containing the recombinant human plasmids harboring the designated sequences were streaked on a LB solid plate containing ampicillin sodium and incubated upside down at 37°C overnight. A single clone was picked from the plate and expanded in 200 mL of LB medium containing ampicillin sodium (Sigma, Cat. #: L7658). Plasmid extraction was then performed according to the instructions of NucleoBond Xtra Midi EF Kit (MACHEREY-NAGEL, Cat. #: 740420.50). Finally, sufficient quantities of recombinant human plasmids pWX4.1/p28 and pWX4.1/EBI3 were obtained. The pWX4.1/p28 and pWX4.1/EBI3 plasmids were diluted and the concentrations were detected by Nano-Drop (Thermo Scientific, Model No.: ND2000), followed by gene sequencing for verification prior to subsequent transfection and expression.

2) Transient transfection and expression: In accordance with the fermentation platform process of the Protein Science Department of WuXi Biologics, CHO-K1 host cells were diluted to a specified density using the platform process medium provided by the manufacturer. Plasmids pWX4.1/p28 and pWX4.1/EBI3 were mixed uniformly at a mass ratio of 1:1, and the premixed plasmids and the transfection reagent polyethyleneimine (PEI) were added to the CHO-K1 cells to complete transfection (at the amounts specified by the platform). Each host cell was expressed in a 2 L volume. The cells are then cultured on a shaker, with the shaker set to parameters of 36.5°C, 120 RPM, 6% CO₂, and 85% humidity. The day when transfection is completed was designated as Day 0. On Day 1 post-transfection, the shaker parameters were adjusted to 31°C, 120 RPM, 6% CO₂, and 85% humidity, and culture was continued under these shaker parameters until harvest on Day 7 post-transfection. To ensure adequate nutrient supply during cell culture, feed medium and glucose were added to the cells at specified ratios according to the platform process on Day 0 and Day 4 post- transfection. The culture was harvested on Day 7 for downstream purification.

3) Affinity Chromatography (AC): The cell culture harvested on day 7 was centrifuged at 10000×g for 30 minutes using a floor-standing centrifuge. The supernatant was collected and filtered through a 0.22 µm filter membrane to obtain a clarified supernatant containing the target protein. First, 20 mL Ni-Excel was used to capture the target protein; and particularly, the AKTA chromatography system was used. The sample was loaded first at a flow rate of 20 mL/min. 50 mM PB, 200 mM NaCl, 10 mM imidazole (pH 8.0) was used as the EQ (equilibration) buffer, and the column was equilibrated with five column volumes of EQ buffer until baseline stabilization. 50 mM PB, 200 mM NaCl, 10 mM imidazole, pH 8.0, 0.1% TX114 (V/V) was used as Wash2 (washing buffer), and the column was washed with ten column volumes of Wash2 for no less than 60 minutes to remove endotoxins, followed by re-equilibration with seven column volumes of EQ buffer. 50 mM PB, 200 mM NaCl, 400 mM imidazole, pH 8.0 was used as elution buffer to elute the target protein at a flow rate of 2.0 mL/min over three column volumes. The column was subsequently regenerated with 0.5 M NaOH at a flow rate of 5 mL/min for 15 minutes, followed by re-equilibration with ten column volumes of EQ buffer until AKTA baseline stabilization. The eluate was collected as the target protein obtained from the first step of affinity chromatography, and analyzed by SDS-PAGE.

4) Size Exclusion Chromatography (SEC): To remove aggregates and part of the impurity proteins from the sample eluted by affinity chromatography, size exclusion chromatography for purification was employed in the present study. PBS at pH 7.4 was selected as the equilibration buffer (Buffer A). First, the IL-27 protein solution obtained after affinity chromatography was loaded onto a 320 mL HiLoad 26/600 Superdex 200 pg column at a flow rate of 2.5 mL/min. After the sample loading was completed, the target protein peak was appeared gradually. The target protein was collected to obtain purified IL-27. SDS-PAGE analysis indicated a high degree of purity, although minor impurity proteins still existed.

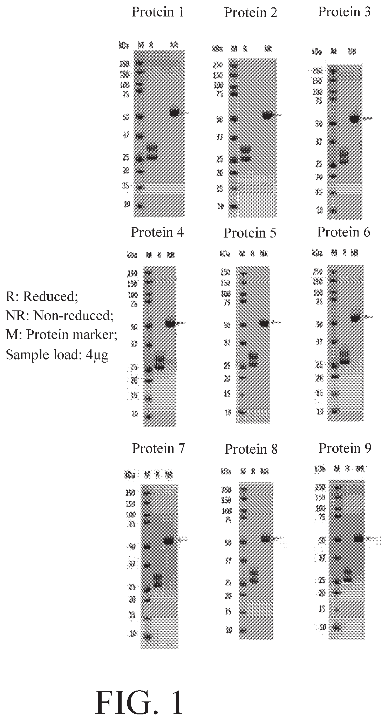

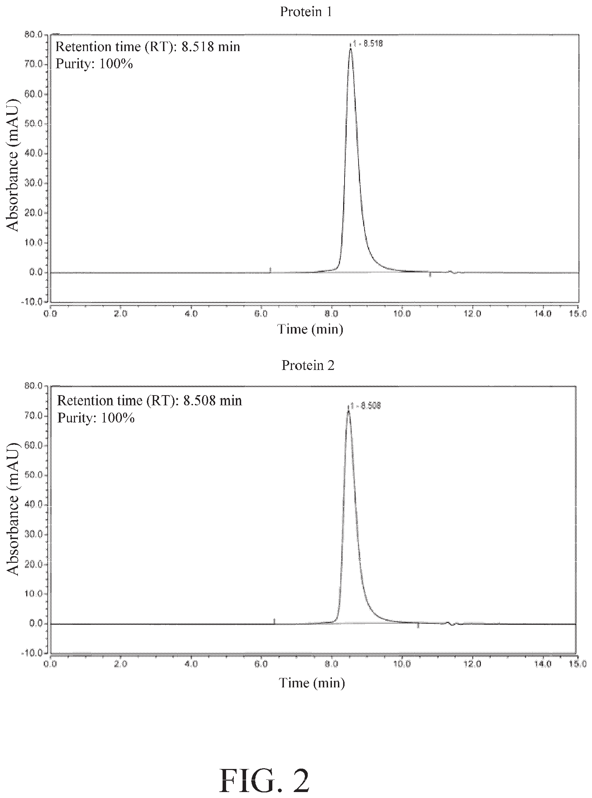

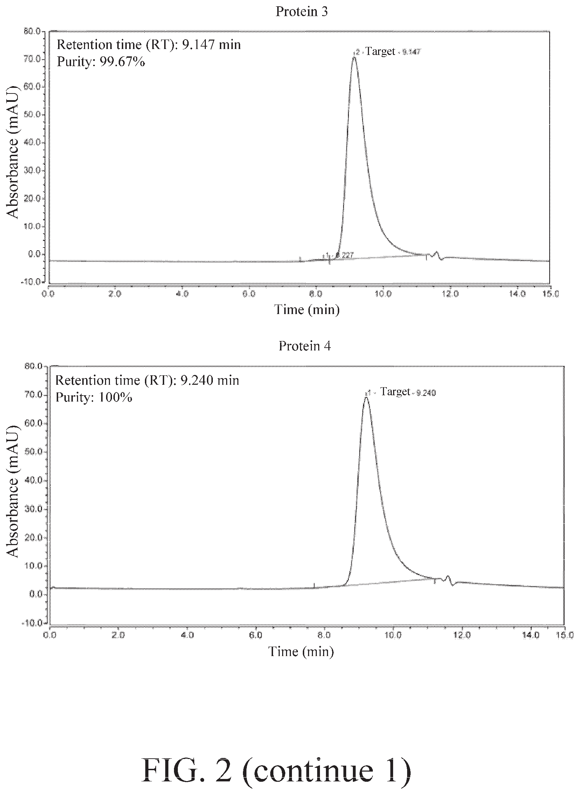

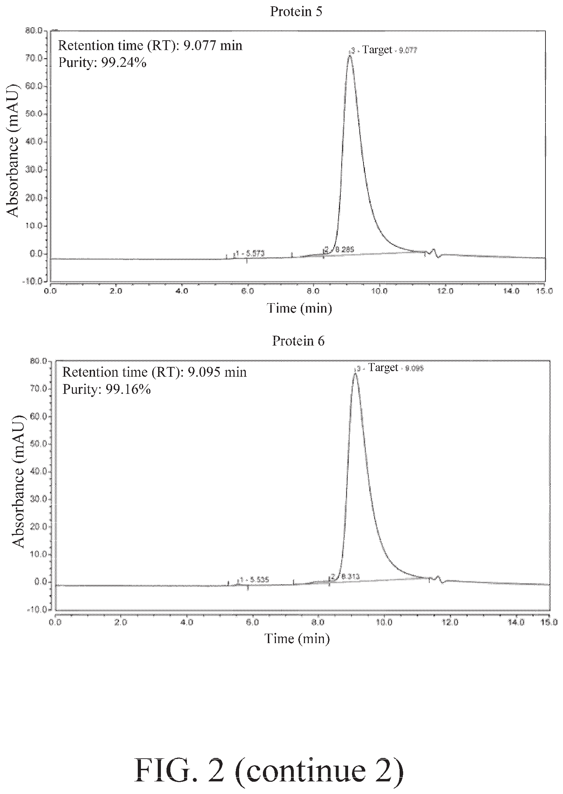

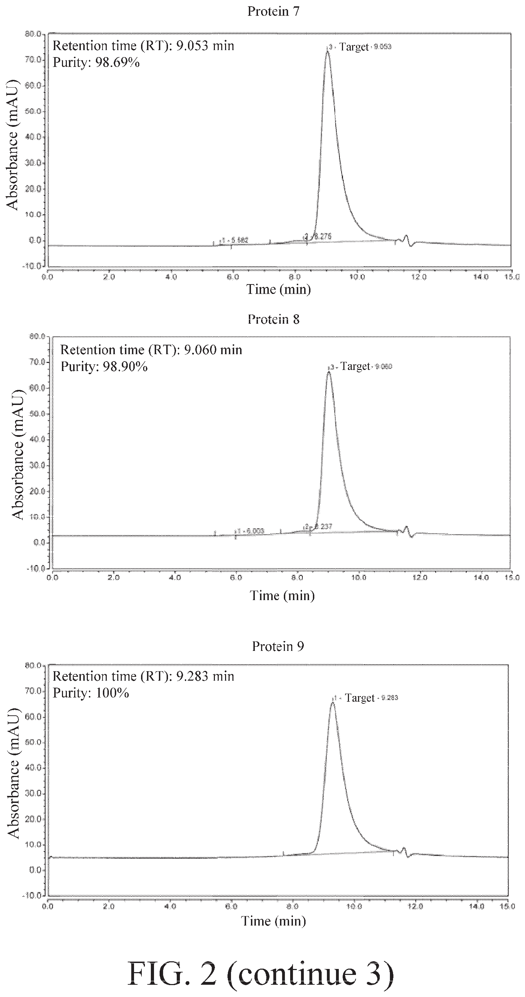

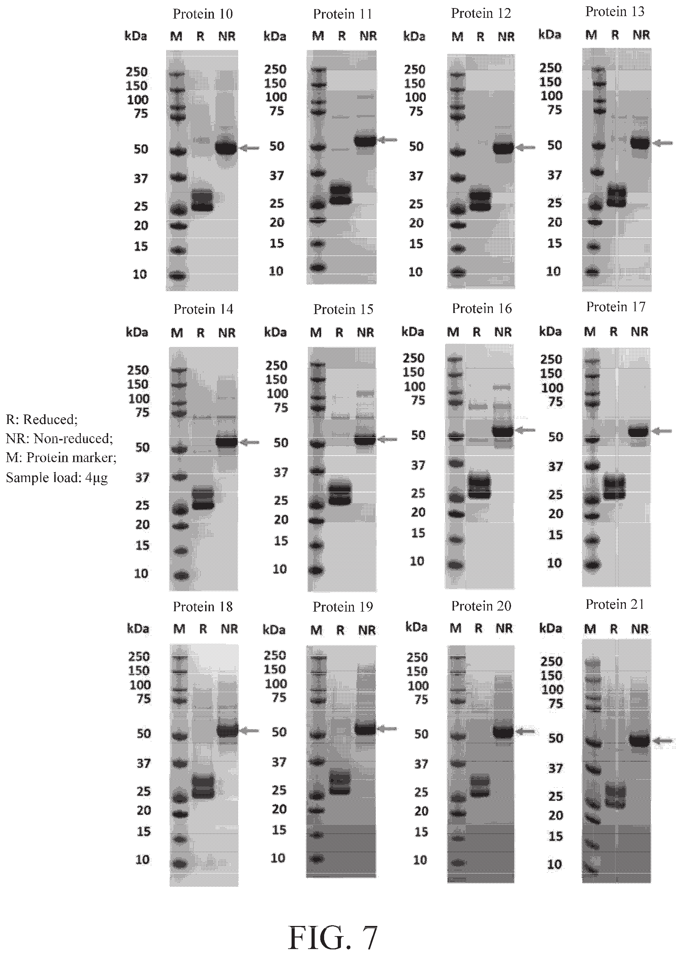

5) Further polishing purification by SP-HP: To further polishing-purify and obtain purified IL-27 protein, the ion exchange chromatography using an SP-HP column for purification was employed in the present study. 20 mM NaAC, pH 5.5 was selected as the equilibration buffer (Buffer A), and 20 mM NaAC, pH 5.5, 1 M NaCl was selected as the elution buffer (Buffer B). First, the SEC-purified IL-27 protein solution was diluted ten-fold with Buffer A to reduce the conductivity to below 8 mS, and the diluted sample was then loaded onto a 5 mL SP-HP column at a flow rate of 5 mL/min. After the sample loading was completed, a gradient elution was performed with Buffer B, with the proportion of Buffer B adjusted from 40% to 80% over 25 column volumes at an elution flow rate of 2.5 mL/min. The eluted protein was collected to obtain purified IL-27. SDS-PAGE gel analysis diagrams (as shown in Figure 1 and Figure 7) demonstrated a substantial improvement in IL-27 purity following purification, and HPLC results confirmed a protein purity of 100% (as shown in Figure 2).

6) Size Exclusion Chromatography (SEC): To further remove host proteins from the sample, size exclusion chromatography was used in this study for polishing purification. PBS, pH 7.4 was selected as the equilibration buffer (Buffer A). The IL-27 protein solution was loaded onto a 120 mL HiLoad 16/600 Superdex 200 pg column at a flow rate of 1.0 mL/min. After the sample loading was completed, the target protein was collected to obtain purified IL-27.

7) LC-MS Verification: The molecular weight of the purified IL-27 was confirmed by LC-MS. The theoretical molecular weights of proteins and the molecular weights measured by LC-MS analysis are shown in the table below. The measured molecular weights were consistent with the theoretical molecular weights of proteins, confirming that all samples are IL-27 proteins.

**Table 3: Results of Measured Molecular Weights of Proteins**

| Protein No. | Theoretical molecular weight (Da) | Measured molecular weight (Da) |
|---|---|---|
| Protein1 | 49196.70 | 49199.04 |
| Protein2 | 49124.57 | 49127.47 |
| Protein3 | 49126.14 | 49129.31 |
| Protein4 | 49111.17 | 49113.78 |
| Protein5 | 49168.33 | 49170.81 |
| Protein6 | 49155.29 | 49157.82 |
| Protein7 | 49417.46 | 49419.97 |
| Protein8 | 49401.42 | 49403.68 |
| Protein9 | 49127.28 | 49130.75 |

8) Host Protein Detection: The purpose of this experiment was to detect residual host cell proteins (HCPs) in IL-27 protein. A double-antibody one-step sandwich enzyme-linked immunosorbent assay (ELISA) was performed using a kit (Cygnus, F550-1). Samples, standards, and HRP-labeled detection antibodies were sequentially added to microplate wells pre-coated with anti-CHO host cell residual protein (CHO-HCP) antibodies, followed by incubation and thorough washing. Color development was performed with the substrate TMB, and TMB was converted to blue under the catalysis by peroxidase and subsequently converted to the final yellow color upon acidification. The intensity of the color is positively correlated with the residual CHO host cell protein (CHO-HCP) in the sample. The absorbance (OD value) was measured at a wavelength of 450 nm using a microplate reader, and sample concentration were calculated. Table 4 provides the results of protein concentration analysis results.

**Table 4 Host Protein Analysis and Detection Results**

| Sample ID | Final HCP ratio result (ppm) |
|---|---|
| Protein1 | 2146 |
| Protein2 | 1330 |
| Protein3 | 722 |
| Protein4 | 1115 |
| Protein5 | 4869 |
| Protein6 | 2091 |
| Protein7 | 2871 |
| Protein8 | 5667 |
| Protein9 | 5519 |

9) ELISA Assay: The purpose of the study was to detect the binding activity of human IL-27 protein samples and human IL-27RA-Fc protein (Sino Biological, Cat. #:10489-H02H). The experimental methods were as follows:
i) Coating: IL27-His protein reference substance (Cat. #: 10076-H08S) and Proteins 1-9 were coated at 2 µg/mL, 100 mL/ well, and incubated overnight at 4°C;
ii) Blocking: The liquid in the plate was discarded and the plate was blotted dry, 2% BSA blocking buffer was added at 300 µL/well, and the plate was sealed and incubated at room temperature for 1 hour;
iii) Plate Washing: The plate was washed 2 times with PBS-T Phosphate-Tween washing solution at 300 µL/well, and blotted dry after the last wash;
iv) Sample Dilution: IL27RA-Fc protein (Cat. #: 10489-H02H) was diluted to 20000 ng/mL using sample diluent (0.1% BSA dissolved in PBST), followed by serial dilutions as set forth in the table below, with thorough mixing performed after each dilution step.

**Table 5 Sample Dilution**

| Sample No. | Sample volume (µL) | Volume of sample diluent added (µL) | Final concentration (ng/mL) |
|---|---|---|---|
| A | 200 | 800 | 4000 |
| B | 200 | 800 | 800 |
| C | 200 | 800 | 160 |
| D | 200 | 800 | 32 |
| E | 200 | 800 | 6.4 |
| F | 200 | 800 | 1.28 |
| / | / | 800 | 0 |

v) Plate Washing: The plate was washed 3 times with 300 µL/well of PBST washing solution, and blotted dry after the last wash.
vi) Secondary antibody Addition: The secondary antibody, goat anti-human IgG (Fc)/HRP was diluted to 0.1 µg/mL mixed thoroughly, and added at 100 µL/well, followed by incubation at room temperature for 1 h;
vii) Plate Washing: Same as step v);
viii) Color Development: Solution A and solution B at a ratio of 1:1 were mixed, and 200 µL was added to each well, followed by incubation at room temperature protected from light for 15 minutes;
ix) Termination: 50 µL of stop solution was added to each well, and the OD value was immediately measured at a wavelength of 450 nm.

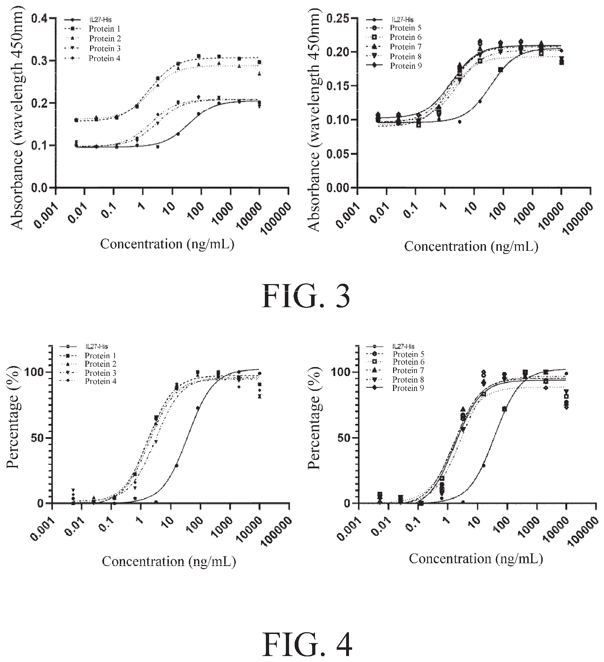

The results shown in Table 6 below demonstrate that, based on the binding activity data, it can be concluded that Proteins 1-9 exhibited favorable binding activity to human IL27RA-Fc protein (as shown in Figures 3 and 4).

**Table 6 Binding Activity Data**

| Sample | Cat. #: | Lot | binding protein | Cat. #: | Lot | EC50(ng/mL) |
|---|---|---|---|---|---|---|
| IL27-His | 10076-H08S | MA17M Y0822 | IL27RA -Fc | 10489-H02H | MA16AU190 3 | 85.875 (the mean value of the control group obtained from multiple experiments) |
| Protein1 | / | / | | | | 179.4 |
| Protein2 | / | / | | | | 331.3 |
| Protein3 | / | / | | | | 147.3 |
| Protein4 | / | / | | | | 89.2 |
| Protein5 | / | / | | | | 157.9 |
| Protein6 | / | / | | | | 73.0 |
| Protein7 | / | / | | | | 126.3 |
| Protein8 | / | / | | | | 367.6 |
| Protein9 | / | / | | | | 138.1 |
| Protein10 | | | | | | 46.5 |
| Protein11 | | | | | | 49.8 |
| Protein12 | | | | | | 53.1 |
| Protein13 | | | | | | 77.4 |
| Protein14 | | | | | | 51.6 |
| Protein15 | | | | | | 55.4 |
| Protein16 | | | | | | 71.3 |
| Protein17 | | | | | | 42.3 |
| Protein18 | | | | | | 104.5 |
| Protein20 | | | | | | 53.0 |
| Protein21 | | | | | | 66.4 |

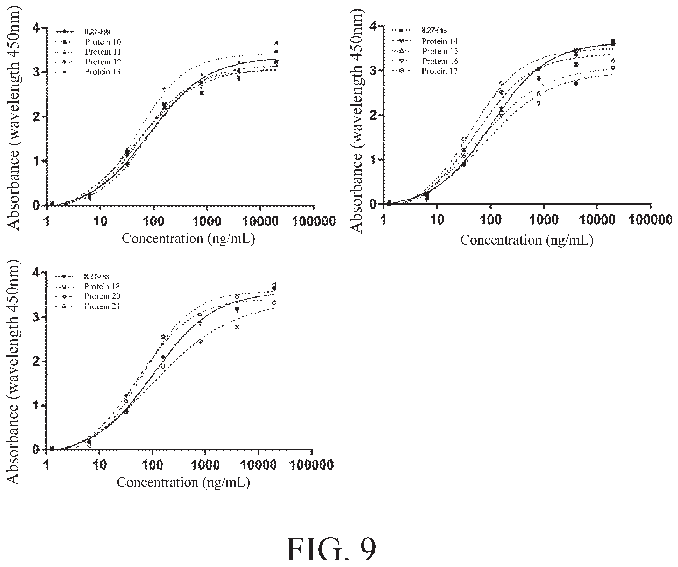

Figure 9 shows the test results for the binding activity of recombinant IL-27 Proteins 10-21 to human IL27RA-Fc. In addition, as directly demonstrated by the data in Table 6 above, Proteins 10-18 and 20-21 exhibited favorable binding activity to human IL27RA-Fc protein.

### Example 2: Activity Assay of Recombinantly Expressed IL-27

In this Example, the activity of IL-27 was determined by cytopathic effect inhibition assay (Seita J, et al. (2008) Interleukin-27 directly induces differentiation in hematopoietic stem cells. Blood. 111(4): 1903-12). The experimental methods were as follows:
1. Cell Culture: HepG2 cells were expanded to the required cell amount (1.92 × 10⁶ cells);
2. Cell Seeding: HepG2 cells were harvested, adjusted to the required cell density, and seeded into a 96-well plate; upon completion, the 96-well plate was placed in a humidified incubator at 37°Cwith 5% CO₂;
3. Sample Addition: Samples were serially diluted in 5-fold increments for a total of 10 dilutions, with duplicate wells for each dilution; the samples were added and incubated overnight;
4. Virus Addition: VSV virus was diluted to certain fold and added to a 96-well plate, followed by incubation overnight;
5. Detection: MTT was added, and after 4 hours of incubation, triple solution was added; the plate was incubated overnight in a humified incubator at 37°C with 5% CO₂, and the absorbance was read using a microplate reader;

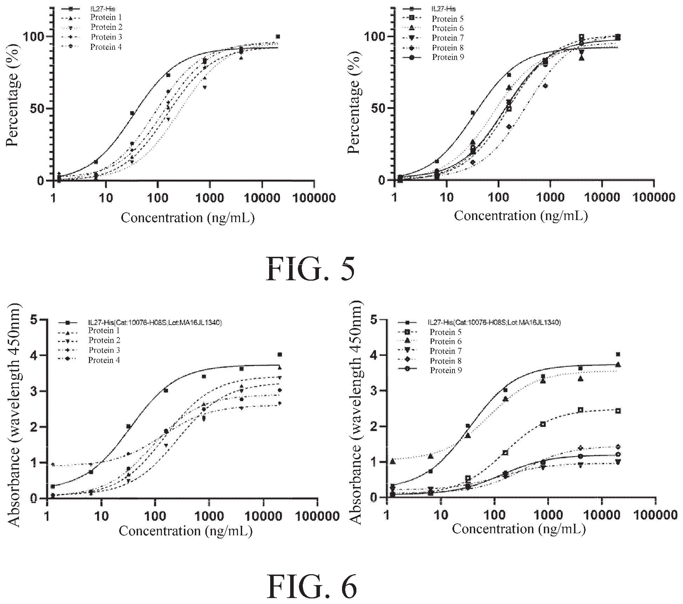

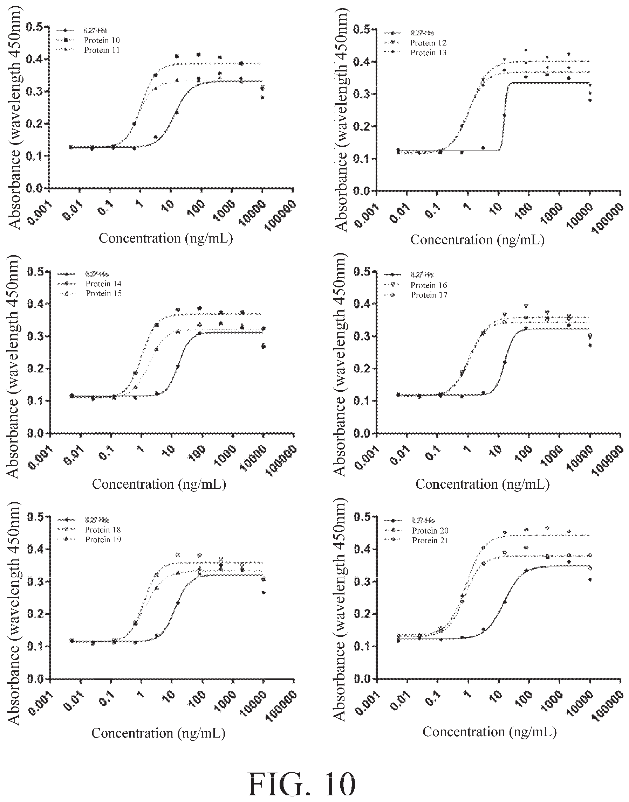

Using HepG2 as the test cells, samples at different concentrations were incubated with the cells, followed by viral challenge to induce cytopathic effect. The OD values of the cells were determined by MTT assay. The results are shown in Figures 5, 6 and 10, and Table 7. Comparison with the positive control demonstrated that the samples exhibited a significant protective effect on cells, demonstrating that Proteins 1-9 and 10-21 possess biological activity capable of inhibiting cytopathic effect in HepG2 cells.

**Table 7 Summary of Activity Results**

| Cat. #: | Sample | Lot | EC₅₀(ng/mL) |
|---|---|---|---|
| 10076-H08S | IL27-His | MA17MY0822 | 14.85(the mean value of the control group obtained from multiple experiments) |
| | | Protein1 | 1.7 |
| | | Protein2 | 1.9 |
| | | Protein3 | 3.3 |
| | | Protein4 | 2 |
| | | Protein5 | 1.9 |
| | | Protein6 | 1.6 |
| | | Protein7 | 1.7 |
| | | Protein8 | 2.7 |
| | | Protein9 | 2.1 |
| | | Protein10 | 1.1 |
| | | Protein11 | 0.9 |
| | | Protein12 | 1.2 |
| | | Protein13 | 1 |
| | | Protein14 | 1 |
| | | Protein15 | 1.8 |
| | | Protein16 | 1.2 |
| | | Protein17 | 1 |
| | | Protein18 | 1.2 |
| | | Protein19 | 1.4 |
| | | Protein20 | 0.8 |
| | | Protein21 | 0.8 |

The results in Table 7 clearly demonstrate that Proteins 1-21 exhibit substantially superior compared to commercially available recombinant IL-27 protein.

All publications, patents, patent applications or other references cited herein are hereby incorporated by reference in their entirety for all purposes, to the same extent as if each individual publication, patent, patent application or other reference were individually indicated to be incorporated by reference for all purposes. Amino acid sequence

| SEQ ID NO | Name | sequence |
|---|---|---|
| 1 | p28_S91C | MGWSCIILFLVATATGVHSFPRPPGRPQLSLQELRREFTVSLHLARKLLS EVRGQAHRFAESHLPGVNLYLLPLGEQLPDVCLTFQAWRRLSDPERLC FISTTLQPFHALLGGLGTQGRWTNMERMQLWAMRLDLRDLQRHLRFQ VLAAGFNLPEEEEEEEEEEEEEERKGLLPGALGSALQGPAQVSWPQLLST YRLLHSLELVLSRAVRELLLLSKAGHSVWPLGFPTLSPQPHHHHHH |
| 2 | EBI3_S98C | MGWSCIILFLVATATGVHSRKGPPAALTLPRVQCRASRYPIAVDCSWTL PPAPNSTSPVSFIATYRLGMAARGHSWPCLQQTPTSTSCTITDVQLFCM APYVLNVTAVHPWGSSSSFVPFITEHIIKPDPPEGVRLSPLAERQLQVQW EPPGSWPFPEIFSLKYWIRYKRQGAARFHRVGPIEATSFILRAVRPRARY YVQVAAQDLTDYGELSDWSLPATATMSLGK |
| 3 | p28_D89C | MGWSCIILFLVATATGVHSFPRPPGRPQLSLQELRREFTVSLHLARKLLS EVRGQAHRFAESHLPGVNLYLLPLGEQLPCVSLTFQAWRRLSDPERLC FISTTLQPFHALLGGLGTQGRWTNMERMQLWAMRLDLRDLQRHLRFQ VLAAGFNLPEEEEEEEEEEEEERKGLLPGALGSALQGPAQVSWPQLLST YRLLHSLELVLSRAVRELLLLSKAGHSVWPLGFPTLSPQPHHHHHH |
| 4 | EBI3_M99C | MGWSCIILFLVATATGVHSRKGPPAALTLPRVQCRASRYPIAVDCSWTL PPAPNSTSPVSFIATYRLGMAARGHSWPCLQQTPTSTSCTITDVQLFSCA PYVLNVTAVHPWGSSSSFVPFITEHIIKPDPPEGVRLSPLAERQLQVQWE PPGSWPFPEIFSLKYWIRYKRQGAARFHRVGPIEATSFILRAVRPRARYY VQVAAQDLTDYGELSDWSLPATATMSLGK |
| 5 | p28_L83C | MGWSCIILFLVATATGVHSFPRPPGRPQLSLQELRREFTVSLHLARKLLS EVRGQAHRFAESHLPGVNLYLLPCGEQLPDVSLTFQAWRRLSDPERLC FISTTLQPFHALLGGLGTQGRWTNMERMQLWAMRLDLRDLQRHLRFQ VLAAGFNLPEEEEEEEEEEEEEERKGLLPGALGSALQGPAQVSWPQLLST YRLLHSLELVLSRAVRELLLLSKAGHSVWPLGFPTLSPQPHHHHHH |
| 6 | EBI3_Q95C | MGWSCIILFLVATATGVHSRKGPPAALTLPRVQCRASRYPIAVDCSWTL PPAPNSTSPVSFIATYRLGMAARGHSWPCLQQTPTSTSCTITDVCLFSM APYVLNVTAVHPWGSSSSFVPFITEHIIKPDPPEGVRLSPLAERQLQVQW EPPGSWPFPEIFSLKYWIRYKRQGAARFHRVGPIEATSFILRAVRPRARY YVQVAAQDLTDYGELSDWSLPATATMSLGK |
| 7 | p28_Q86C | MGWSCIILFLVATATGVHSFPRPPGRPQLSLQELRREFTVSLHLARKLLS EVRGQAHRFAESHLPGVNLYLLPLGECLPDVSLTFQAWRRLSDPERLC FISTTLQPFHALLGGLGTQGRWTNMERMQLWAMRLDLRDLQRHLRFQ VLAAGFNLPEEEEEEEEEEEEEERKGLLPGALGSALQGPAQVSWPQLLST YRLLHSLELVLSRAVRELLLLSKAGHSVWPLGFPTLSPQPHHHHHH |
| 8 | EBI3_P41C | MGWSCIILFLVATATGVHSRKGPPAALTLPRVQCRASRYCIAVDCSWT LPPAPNSTSPVSFIATYRLGMAARGHSWPCLQQTPTSTSCTITDVQLFSM |
| | | APYVLNVTAVHPWGSSSSFVPFITEHIIKPDPPEGVRLSPLAERQLQVQW EPPGSWPFPEIFSLKYWIRYKRQGAARFHRVGPIEATSFILRAVRPRARY YVQVAAQDLTDYGELSDWSLPATATMSLGK |
| 9 | p28_F94C | MGWSCIILFLVATATGVHSFPRPPGRPQLSLQELRREFTVSLHLARKLLS EVRGQAHRFAESHLPGVNLYLLPLGEQLPDVSLTCQAWRRLSDPERLC FISTTLQPFHALLGGLGTQGRWTNMERMQLWAMRLDLRDLQRHLRFQ VLAAGFNLPEEEEEEEEEEEEEERKGLLPGALGSALQGPAQVSWPQLLST YRLLHSLELVLSRAVRELLLLSKAGHSVWPLGFPTLSPQPHHHHHH |
| 10 | EBI3_L96C | MGWSCIILFLVATATGVHSRKGPPAALTLPRVQCRASRYPIAVDCSWTL PPAPNSTSPVSFIATYRLGMAARGHSWPCLQQTPTSTSCTITDVQCFSM APYVLNVTAVHPWGSSSSFVPFITEHIIKPDPPEGVRLSPLAERQLQVQW EPPGSWPFPEIFSLKYWIRYKRQGAARFHRVGPIEATSFILRAVRPRARY YVQVAAQDLTDYGELSDWSLPATATMSLGK |
| 11 | p28_Q95C | MGWSCIILFLVATATGVHSFPRPPGRPQLSLQELRREFTVSLHLARKLLS EVRGQAHRFAESHLPGVNLYLLPLGEQLPDVSLTFCAWRRLSDPERLC FISTTLQPFHALLGGLGTQGRWTNMERMQLWAMRLDLRDLQRHLRFQ VLAAGFNLPEEEEEEEEEEEEEEERKGLLPGALGSALQGPAQVSWPQLLST YRLLHSLELVLSRAVRELLLLSKAGHSVWPLGFPTLSPQPHHHHHH |
| 12 | EBI3_D93C | MGWSCIILFLVATATGVHSRKGPPAALTLPRVQCRASRYPIAVDCSWTL PPAPNSTSPVSFIATYRLGMAARGHSWPCLQQTPTSTSCTITCVQLFSM APYVLNVTAVHPWGSSSSFVPFITEHIIKPDPPEGVRLSPLAERQLQVQW EPPGSWPFPEIFSLKYWIRYKRQGAARFHRVGPIEATSFILRAVRPRARY YVQVAAQDLTDYGELSDWSLPATATMSLGK |
| 13 | p28_ L80C | MGWSCIILFLVATATGVHSFPRPPGRPQLSLQELRREFTVSLHLARKLLS EVRGQAHRFAESHLPGVNLYCLPLGEQLPDVSLTFQAWRRLSDPERLC FISTTLQPFHALLGGLGTQGRWTNMERMQLWAMRLDLRDLQRHLRFQ VLAAGFNLPEEEEEEEEEEEEEERKGLLPGALGSALQGPAQVSWPQLLST YRLLHSLELVLSRAVRELLLLSKAGHSVWPLGFPTLSPQPHHHHHH |
| 14 | EBI3_G69C | MGWSCIILFLVATATGVHSRKGPPAALTLPRVQCRASRYPIAVDCSWTL PPAPNSTSPVSFIATYRLCMAARGHSWPCLQQTPTSTSCTITDVQLFSM APYVLNVTAVHPWGSSSSFVPFITEHIIKPDPPEGVRLSPLAERQLQVQW EPPGSWPFPEIFSLKYWIRYKRQGAARFHRVGPIEATSFILRAVRPRARY YVQVAAQDLTDYGELSDWSLPATATMSLGK |
| 15 | p28_ P82C | MGWSCIILFLVATATGVHSFPRPPGRPQLSLQELRREFTVSLHLARKLLS EVRGQAHRFAESHLPGVNLYLLCLGEQLPDVSLTFQAWRRLSDPERLC FISTTLQPFHALLGGLGTQGRWTNMERMQLWAMRLDLRDLQRHLRFQ VLAAGFNLPEEEEEEEEEEEEEERKGLLPGALGSALQGPAQVSWPQLLST YRLLHSLELVLSRAVRELLLLSKAGHSVWPLGFPTLSPQPHHHHHH |
| 16 | EBI3_ M70C | MGWSCIILFLVATATGVHSRKGPPAALTLPRVQCRASRYPIAVDCSWTL PPAPNSTSPVSFIATYRLGCAARGHSWPCLQQTPTSTSCTITDVQLFSMA PYVLNVTAVHPWGSSSSFVPFITEHIIKPDPPEGVRLSPLAERQLQVQWE PPGSWPFPEIFSLKYWIRYKRQGAARFHRVGPIEATSFILRAVRPRARYY VQVAAQDLTDYGELSDWSLPATATMSLGK |
| 17 | p28_ G84C | MGWSCIILFLVATATGVHSFPRPPGRPQLSLQELRREFTVSLHLARKLLS EVRGQAHRFAESHLPGVNLYLLPLCEQLPDVSLTFQAWRRLSDPERLC FISTTLQPFHALLGGLGTQGRWTNMERMQLWAMRLDLRDLQRHLRFQ VLAAGFNLPEEEEEEEEEEEEEERKGLLPGALGSALQGPAQVSWPQLLST YRLLHSLELVLSRAVRELLLLSKAGHSVWPLGFPTLSPQPHHHHHH |
| 18 | EBI3_ A100C | MGWSCIILFLVATATGVHSRKGPPAALTLPRVQCRASRYPIAVDCSWTL PPAPNSTSPVSFIATYRLGMAARGHSWPCLQQTPTSTSCTITDVQLFSM CPYVLNVTAVHPWGSSSSFVPFITEHIIKPDPPEGVRLSPLAERQLQVQW EPPGSWPFPEIFSLKYWIRYKRQGAARFHRVGPIEATSFILRAVRPRARY YVQVAAQDLTDYGELSDWSLPATATMSLGK |
| 19 | p28_ E85C | MGWSCIILFLVATATGVHSFPRPPGRPQLSLQELRREFTVSLHLARKLLS EVRGQAHRFAESHLPGVNLYLLPLGCQLPDVSLTFQAWRRLSDPERLC FISTTLQPFHALLGGLGTQGRWTNMERMQLWAMRLDLRDLQRHLRFQ VLAAGFNLPEEEEEEEEEEEEEERKGLLPGALGSALQGPAQVSWPQLLST YRLLHSLELVLSRAVRELLLLSKAGHSVWPLGFPTLSPQPHHHHHH |
| 20 | EBI3_ P101C | MGWSCIILFLVATATGVHSRKGPPAALTLPRVQCRASRYPIAVDCSWTL PPAPNSTSPVSFIATYRLGMAARGHSWPCLQQTPTSTSCTITDVQLFSM CPYVLNVTAVHPWGSSSSFVPFITEHIIKPDPPEGVRLSPLAERQLQVQW EPPGSWPFPEIFSLKYWIRYKRQGAARFHRVGPIEATSFILRAVRPRARY YVQVAAQDLTDYGELSDWSLPATATMSLGK |
| 21 | p28_ L87C | MGWSCIILFLVATATGVHSFPRPPGRPQLSLQELRREFTVSLHLARKLLS EVRGQAHRFAESHLPGVNLYLLPLGEQCPDVSLTFQAWRRLSDPERLC FISTTLQPFHALLGGLGTQGRWTNMERMQLWAMRLDLRDLQRHLRFQ VLAAGFNLPEEEEEEEEEEEEEERKGLLPGALGSALQGPAQVSWPQLLST YRLLHSLELVLSRAVRELLLLSKAGHSVWPLGFPTLSPQPHHHHHH |
| 22 | EBI3_ P120C | MGWSCIILFLVATATGVHSRKGPPAALTLPRVQCRASRYPIAVDCSWTL PPAPNSTSPVSFIATYRLGMAARGHSWPCLQQTPTSTSCTITDVQLFSM APYVLNVTAVHPWGSSSSFVCFITEHIIKPDPPEGVRLSPLAERQLQVQ WEPPGSWPFPEIFSLKYWIRYKRQGAARFHRVGPIEATSFILRAVRPRAR YYVQVAAQDLTDYGELSDWSLPATATMSLGK |
| 23 | p28_ L81C | MGWSCIILFLVATATGVHSFPRPPGRPQLSLQELRREFTVSLHLARKLLS EVRGQAHRFAESHLPGVNLYLCPLGEQLPDVSLTFQAWRRLSDPERLC FISTTLQPFHALLGGLGTQGRWTNMERMQLWAMRLDLRDLQRHLRFQ VLAAGFNLPEEEEEEEEEEEEEERKGLLPGALGSALQGPAQVSWPQLLST YRLLHSLELVLSRAVRELLLLSKAGHSVWPLGFPTLSPQPHHHHHH |
| 24 | Signal peptide sequence | MGWSCIILFLVATATGVHS |
| 25 | p28_ Y79C | MGWSCIILFLVATATGVHSFPRPPGRPQLSLQELRREFTVSLHLARKLLS EVRGQAHRFAESHLPGVNLCLLPLGEQLPDVSLTFQAWRRLSDPERLC FISTTLQPFHALLGGLGTQGRWTNMERMQLWAMRLDLRDLQRHLRFQ VLAAGFNLPEEEEEEEEEEEEEERKGLLPGALGSALQGPAQVSWPQLLST YRLLHSLELVLSRAVRELLLLSKAGHSVWPLGFPTLSPQPHHHHHH |
| 26 | wild-type p28 subunit amino acid sequence | MGQTAGDLGWRLSLLLLPLLLVQAGVWGFPRPPGRPQLSLQELRREFT VSLHLARKLLSEVRGQAHRFAESHLPGVNLYLLPLGEQLPDVSLTFQA WRRLSDPERLCFISTTLQPFHALLGGLGTQGRWTNMERMQLWAMRLD LRDLQRHLRFQVLAAGFNLPEEEEEEEEEEEEEERKGLLPGALGSALQGP |
| | | AQVSWPQLLSTYRLLHSLELVLSRAVRELLLLSKAGHSVWPLGFPTLSP QP |
| 27 | wild-type EBI/3 amino acid sequence | MTPQLLLALVLWASCPPCSGRKGPPAALTLPRVQCRASRYPIAVDCSW TLPPAPNSTSPVSFIATYRLGMAARGHSWPCLQQTPTSTSCTITDVQLFS MAPYVLNVTAVHPWGSSSSFVPFITEHIIKPDPPEGVRLSPLAERQLQVQ WEPPGSWPFPEIFSLKYWIRYKRQGAARFHRVGPIEATSFILRAVRPRAR YYVQVAAQDLTDYGELSDWSLPATATMSLGK |

## Claims

1. A p28 subunit of a recombinantIL-27 protein, comprising an amino acid sequence selected from any one of SEQ ID NO: 1, 3, 5, 7, 9, 11, 13, 15, 17, 19, 21, 23, and 25.

2. An EBI3 subunit of a recombinant IL-27 protein, comprising an amino acid sequence selected from any one of SEQ ID NO: 2, 4, 6, 8, 10, 12, 14, 16, 18, 20, and 22.

3. A recombinant IL-27 protein, comprising a p28 subunit and an EBI3 subunit, wherein the p28 subunit comprises an amino acid sequence selected from any one of SEQ ID NO: 1, 3, 5, 7, 9, 11, 13, 15, 17, 19, 21, 23, 23, and 25, and the EBI3 subunit comprises an amino acid sequence selected from any one of SEQ ID NO: 2, 4, 6, 8, 10, 12, 14, 16, 18, 20, and 22.

4. The recombinant IL-27 protein according to claim 3, wherein the p28 subunit and the EBI3 subunit are selected from any one of the following combinations as designated by SEQ ID NO: SEQ ID NOs: 1 and 2; SEQ ID NOs: 3 and 2; SEQ ID NOs: 3 and 4; SEQ ID NOs: 5 and 4; SEQ ID NOs: 7 and 4;SEQ ID NOs: 17 and 4; SEQ ID NOs: 19 and 4; SEQ ID NOs: 21 and 4; SEQ ID NOs: 1 and 6; SEQ ID NOs: 9 and 8; SEQ ID NOs: 9 and 10; SEQ ID NOs: 11 and 12; SEQ ID NOs: 5 and 14; SEQ ID NOs: 5 and 16; SEQ ID NOs: 13 and 16; SEQ ID NOs: 15 and 16; SEQ ID NOs: 5 and 18; SEQ ID NOs: 23 and 18; SEQ ID NOs: 13 and 20; SEQ ID NOs: 13 and 22; SEQ ID NOs: and 25 and 22.

5. The recombinant IL-27 or one or more subunits thereof according to any one of claims 1, 2 or 3, wherein the sequence further comprises **characterized in that** said sequence further comprises, at the 5' terminus, a signal peptide sequence of MGWSCIILFLVATATGVHS (SEQ ID NO:24).

6. An isolated nucleic acid molecule encoding the recombinant IL-27 protein or one or more subunits thereof of any one of claims 1 to 5.

7. An expression vector comprising and expressing the nucleic acid molecule of claim 6.

8. An isolated host cell comprising the expression vector of claim 7, wherein preferably the cell is a CHO cell.

9. A method for preparing a recombinant IL-27 protein, comprising
introducing a cysteine substitution into a p28 subunit of a recombinant IL-27 protein, wherein the p28 subunit having the amino acid sequence of 
MGQTAGDLGWRLSLLLLPLLLVQAGVWGFPRPPGRPQLSLQELRREFTVSLHLARKLLS
EVRGQAHRFAESHLPGVNLYLLPLGEQLPDVSLTFQAWRRLSDPERLCFISTTLQPFHAL
LGGLGTQGRWTNMERMQLWAMRLDLRDLQRHLRFQVLAAGFNLPEEEEEEEEEEEEE
RKGLLPGALGSALQGPAQVSWPQLLSTYRLLHSLELVLSRAVRELLLLSKAGHSVWPLG
FPTLSPQP (SEQ ID NO:26),
introducing a cysteine substitution into an EBI3 subunit of a recombinant IL-27 protein, wherein the EBI3 subunit having the amino acid sequence of 
MTPQLLLALVLWASCPPCSGRKGPPAALTLPRVQCRASRYPIAVDCSWTLPPAPNSTSPV
SFIATYRLGMAARGHSWPCLQQTPTSTSCTITDVQLFSMAPYVLNVTAVHPWGSSSSFV
PFITEHIIKPDPPEGVRLSPLAERQLQVQWEPPGSWPFPEIFSLKYWIRYKRQGAARFHRV
GPIEATSFILRAVRPRARYYVQVAAQDLTDYGELSDWSLPATATMSLGK (SEQ ID
NO:27),
synthesizing the two modified subunits artificially and assembling them to obtain a recombinant IL-27 protein; or alternatively, obtaining coding sequences of the subunits, transfecting them into an expression vector, expressing them in cells to obtain a recombinant IL-27 protein;
determining the activity of the recombinant IL-27 protein and comparing the activity with that of a wild-type IL-27, thereby obtaining a recombinant IL-27 protein having an activity greater than that of the wild-type IL-27.
